# EUROPÄISCHE PATENTANMELDUNG

(11) **EP 1 053 992 A1**
(43) Veröffentlichungstag der Anmeldung: **22.11.2000**
(21) Anmeldenummer: 00109146.1
(22) Anmeldetag: 05.05.2000
(51) Int. Cl.: C07C 51/285, C07C 55/02, C07C 55/14

(54) **Herstellung von aliphatischen alpha, omega-Dicarbonsäuren**

(30) Priorität: 18.05.1999 DE 19922643
(71) Anmelder: BAYER AG, 51368 Leverkusen (DE)
(72) Erfinder: Schelhaas, Michael, Dr., 50733 Köln (DE); Greiving, Helmut, Dr., Upper St. Clair, PA 15241 (US); Jautelat, Manfred, Dr., 51399 Burscheid (DE)

(57) **Zusammenfassung**

Die vorliegende Erfindung betrifft ein Verfahren zur Herstellung von aliphatischen C₄-C₁₈-α,ω-Dicarbonsäuren aus C₆-C₂₀-α,ω-Dienen mit Wasserstoffperoxid oder Verbindungen, die unter den Reaktionsbedingungen Wasserstoffperoxid freisetzen. Die Reaktion wird in Gegenwart von Übergangsmetallkatalysatoren der Gruppe VI und einer Mischung, welche mindestens eine organische Säure und mindestens ein aprotisches organisches Lösungsmittel enthält durchgeführt.

## Beschreibung

Die vorliegende Erfindung betrifft ein Verfahren zur Herstellung von aliphatischen C₄-C₁₈-α,ω-Dicarbonsäuren aus C₆-C₂₀-α,ω-Dienen.

Aliphatische α,ω-Dicarbonsäuren sind Verbindungen, die aufgrund ihrer Bi-funktionalität eine bedeutende Rolle in der organischen Synthese spielen. Die kurzkettigen Vertreter sind wichtige Reagentien für organische Synthesen aller Art, die höherkettigen Vertreter werden bevorzugt für die Herstellung von Polyamiden, Polyestern, Weichmachern, Alkydharzen oder Schmiermitteln eingesetzt. Eine besondere Bedeutung hat dabei die Adipinsäure, ein wichtiger Ausgangsstoff zur Herstellung von Nylon.

Die technische Herstellung von Adipinsäure verläuft ausgehend von Cyclohexan in einem zweistufigen Prozess. Dabei wird das Cyclohexan zunächst zu einem Gemisch von Cyclohexanol und Cyclohexanon oxidiert. Das erhaltene sogenannte KA-Öl wird in der Regel mit Salpetersäure weiteroxidiert, wobei stöchiometrische Mengen an nitrosen Gasen entstehen. Der alternative Einsatz von Luftsauerstoff als Oxidationsmittel führt zu einer geringeren Produktselektivität.

Ein weiteres Verfahren zur Herstellung von Adipinsäure ist die Biscarbonylierung von Butadien (US 42 59 520), das über die Hydrolyse der Bisester zu Adipinsäure führt. Die Bildung von isomeren Methylglutarat, dem verzweigten Biscarbonylierungs-produkt, lässt sich hierbei nicht unterdrücken.

Für die Herstellung von Adipinsäure aus Cyclohexen wurde das System Wolframsäure/wässrige Wasserstoffperoxidlösung in *tert.*-Butanol eingesetzt. Nachteilig war hierbei vor allem die lange Reaktionsdauer von 24 h. (siehe T. Oguchi et al., *Chem. Lett.* 1989, 857-860).

Es bestand also die Aufgabe, einen leichten und selektiven Zugang zu Adipinsäure zu schaffen, wobei als gut zugängliche Edukte α,ω-Diene eingesetzt werden sollten.

Bekannt ist, dass sich α,ω-Dicarbonsäuren durch oxidative Spaltung von α,ω-Dienen oder cyclischen Alkenen herstellen lassen. Als Oxidationsmittel werden dabei Ozon, Kaliumpermanganat, Chromate oder Peroxide eingesetzt.

Antonelli et al., *J. Org. Chem.* 63, 1998, 7190-7206 beschreiben die Umsetzung von 1,7-Octadien zu Adipinsäure in Gegenwart von wässriger Wasserstoffperoxidlösung und Methyltrioctylammonium-tetrakis-(oxodiperoxowolfram)-phosphat als Phasentransferkatalysator. Mit diesem Verfahren wurde allerdings nur eine Adipinsäure-Ausbeute von 60% erreicht.

Die Lösung der erfindungsgemäßen Aufgabe besteht in einem Verfahren zur Herstellung von gegebenenfalls substituierten aliphatischen C₄-C₁₈-α,ω-Dicarbonsäuren durch Oxidation von gegebenenfalls substituierten C₆-C₂₀-α,ω-Dienen mit Wasserstoffperoxid oder Verbindungen, die unter den Reaktionsbedingungen Wasserstoffperoxid freisetzen, gefunden, das dadurch gekennzeichnet ist, dass die Reaktion in Gegenwart von Übergangsmetallkatalysatoren der Gruppe VI und einer Mischung, welche mindestens eine organische Säure und mindestens ein aprotisches organisches Lösungsmittel enthält, durchgeführt wird.

Durch das erfindungsgemäße Verfahren kann auch Adipinsäure hergestellt werden.

Das erfindungsgemäße Verfahren zeichnet sich dadurch aus, dass innerhalb kurzer Reaktionszeiten die gewünschten Reaktionsprodukte selektiv und in sehr hohen Ausbeuten erhalten werden.

In einer bevorzugten Ausführungsform des erfindungsgemäßen Verfahrens werden als organische Säuren geradkettige oder verzweigte C₂-C₈-Alkylcarbonsäuren, C₆-C₁₄-Arylcarbonsäuren oder C₇-C₁₅-Arylalkylcarbonsäuren eingesetzt, die gegebenenfalls mindestens einen weiteren Substituenten des Typs Halogen, geradkettiges oder verzweigtes C₁-C₆-Alkyl, C₃-C₇-Cycloalkyl oder Carboxyl tragen. Beispiele hierfür sind Essigsäure, Propionsäure, Pivalinsäure, Chloressigsäure, Benzoesäure, Terephthalsäure oder Phenylessigsäure. Besonders bevorzugt wird Essigsäure eingesetzt.

In einer weiteren bevorzugten Ausführungsform des erfindungsgemäßen Verfahrens können auch Mischungen von organischen Säuren eingesetzt werden.

In einer bevorzugten Ausführungsform des erfindungsgemäßen Verfahrens wird als aprotisches organisches Lösungsmittel Dioxan, Ethylacetat, Propylacetat, Butylacetat, Chlorbenzol, Acetonitril oder Benzonitril eingesetzt. Besonders bevorzugt wird Dioxan eingesetzt.

In einer weiteren bevorzugten Ausführungsform des erfindungsgemäßen Verfahrens können auch Mischungen von aprotischen organischen Lösungsmitteln eingesetzt werden.

Bevorzugt wird im erfindungsgemäßen Verfahren die organische Säure im Verhältnis von 1:5 (v/v) bis 5:1 (v/v) zum aprotischem organischen Lösungsmittel eingesetzt, besonders bevorzugt ist ein Verhältnis von 1:2 (v/v) bis 2:1 (v/v), ganz besonders bevorzugt ist ein Verhältnis von 1:1 (v/v).

In einer besonders bevorzugten Ausführungsform des erfindungsgemäßen Verfahrens handelt es sich bei der Mischung aus mindestens einer organischer Säure und mindestens einem aprotischem organischen Lösungsmittel um eine einphasige Mischung.

Als Edukte werden im erfindungsgemäßen Verfahren C₆-C₁₈-α,ω-Diene wie beispielsweise 1,7-Octadien, 1,9-Decadien oder 1,11-Dodecadien eingesetzt, wobei diese gegebenenfalls auch mindestens einen Substituenten, wählbar aus der Reihe C₁-C₆-Alkyl, beispielsweise Methyl, Ethyl, Propyl oder Isopropyl, C₆-C₂₂-Aryl, beispielsweise Phenyl oder Naphthyl, C₇-C₂₄-Arylalkyl, beispielsweise Benzyl oder Phenylethyl oder Halogen, beispielsweise Chlor oder Brom, tragen können. Besonders bevorzugt wird als Edukt 1,7-Octadien eingesetzt, welches durch das erfindungsgemäße Verfahren zu Adipinsäure umgesetzt wird.

In einer weiteren bevorzugten Ausführungsform des erfindungsgemäßen Verfahrens können auch Mischungen von C₆-C₁₈-α,ω-Dienen eingesetzt werden. Die durch das erfindungsgemäße Verfahren erhältlichen C₄-C₁₈-α,ω-Dicarbonsäure-Mischungen eignen sich beispielsweise für die Polyesterherstellung.

Für das erfindungsgemäße Verfahren werden als Übergangsmetallkatalysatoren der Gruppe VI bevorzugt Wolframsäure, Wolframate oder Wolframkomplexe eingesetzt, Besonders bevorzugte Wolframate sind Wolframate der Oxidationsstufen II, IV und VI, ganz besonders bevorzugt werden Na₂WO₄ oder (NH₄)₂WO₄ eingesetzt. Ein bevorzugter Wolframkomplex ist der Peroxokomplex K₂WO₈.

Im erfindungsgemäßen Verfahren wird Wasserstoffperoxid bevorzugt als 5% bis 50% (w/v) wässrige Wasserstoffperoxidlösung eingesetzt, besonders bevorzugt als 30% bis 50% (w/v) wässrige Wasserstoffperoxidlösung.

Als Verbindungen, die unter den Reaktionsbedingungen Wasserstoffperoxid freisetzen, werden bevorzugt Percarbonate, Peroxysäuren oder Perborate verwendet.

Üblicherweise werden im erfindungsgemäßen Verfahren 9 bis 20 Äquivalente Wasserstoffperoxid, bezogen auf das eingesetzte α,ω-Dien, verwendet. Bevorzugt werden 10 bis 13 Äquivalente verwendet.

Üblicherweise wird das erfindungsgemäße Verfahren in einem Temperaturbereich von 40°C bis 150°C durchgeführt, bevorzugt wird bei 50°C bis 120°C gearbeitet.

Besonders bevorzugt wird das erfindungsgemäße Verfahren entlang eines Temperaturprofils durchgeführt. Dabei wird die Temperatur bei Beginn des erfindungsgemäßen Verfahrens im Bereich von 50°C bis 75°C gehalten, nach 30 bis 90 Minuten wird die Reaktionstemperatur auf 85°C bis 120°C erhöht.

In einer bevorzugten Form des erfindungsgemäßen Verfahrens werden die Äquivalente an Wasserstoffperoxid in zwei Stufen zur Reaktionsmischung gegeben. Dabei werden zur Reaktionsmischung zuerst 1,5 bis 3,5 Äquivalente Wasserstoffperoxid, bezogen auf das eingesetzte α,ω-Dien, zugegeben, wobei die Temperatur für 30 bis 90 Minuten in einem Bereich von 50°C bis 75°C gehalten wird. Anschließend wird die Temperatur auf 85°C bis 120°C erhöht und die restlichen 5,5 bis 18,5 Äquivalente Wasserstoffperoxid werden zugegeben. In einer besonders bevorzugten Form des erfindungsgemäßen Verfahrens werden zuerst 2,5 Äquivalente Wasserstoffperoxid bei 65°C zugegeben und die Temperatur wird 60 min gehalten, anschließend wird die Temperatur auf 95°C erhöht und die restlichen 9,5 Äquivalente Wasserstoffperoxid werden zugegeben.

Die in zwei Stufen erfolgende Zugabe an Wasserstoffperoxid und das Temperaturprofil führen zu einer Steigerung der Selektivität bezüglich der Umsetzung zur gewünschten α,ω-Dicarbonsäure.

### Beispiele

Die im folgenden aufgeführten Beispiele sollen die Erfindung illustrieren, ohne sie jedoch auf diese Beispiele zu beschränken.

### Beispiel 1

Zu 40 mmol 1,7-Octadien und 0,78 mmol (NH₄)₂WO₄ in 50 ml Dioxan/ Essigsäure 1:1 (v/v) werden 100 mmol H₂O₂ in Form einer 30% (w/v) wässrigen Lösung gegeben und unter Rühren auf 65°C erwärmt. Nach 60 min wird auf 95°C erhitzt und weitere 380 mmol H₂O₂ werden zugegeben. Nach 12 h wird die Reaktion abgebrochen, Mangandioxid zur katalytischen Zersetzung überschüssigen Peroxides zugesetzt und das Lösungsmittel abdestilliert.

Aus dem Rückstand wird nach Kristallisation 5,55 g Adipinsäure (95% der Theorie) als weißer Feststoff isoliert. Der Anteil Glutarsäure liegt unter 4 %.

### Vergleichsbeispiel 2

Zu 40 mmol 1,7-Octadien und 0,78 mmol (NH₄)₂WO₄ in 50 ml Dioxan werden 100 mmol H₂O₂ in Form einer 30% (w/v) wässrigen Lösung gegeben und unter Rühren auf 65°C erwärmt. Nach 60 min wird auf 95°C erhitzt und weitere 380 mmol H₂O₂ werden zugegeben. Nach 12 h wird die Reaktion abgebrochen, Mangandioxid zur katalytischen Zersetzung überschüssigen Peroxides zugesetzt und das Lösungsmittel abdestilliert.

Aus dem Rückstand wird nach Kristallisation 2,97 g Adipinsäure (51% der Theorie) als weißer Feststoff isoliert.

### Vergleichsbeispiel 3

Die Durchführung erfolgt analog zu Vergleichsbeispiel 2, statt Dioxan wird aber Essigsäure verwendet. Die Ausbeute beträgt nach 24 h Reaktionszeit 3,96 g Adipinsäure (75 % der Theorie).

### Vergleichsbeispiel 4

In einem 100 ml Zweihalskolben werden 40 mmol 1,7-Octadien, 0,78 mmol (NH₄)₂WO₄ und 0,1 mmol Methyltrioctylammoniumhydrogensulfat als Phasentransferkatalysator in 40 ml einer 30% (w/v) Wasserstoffperoxidlösung gelöst, unter Rühren für 30 min bei 75°C gehalten und für 47 h bei 95°C gerührt. Nach abdestillieren des Lösungsmittels werden aus dem Rückstand 4,40 g weißer Feststoff isoliert, der 3,78 g Adipinsäure (65% der Theorie) neben 0.62 g Glutarsäure (Verhältnis Adipinsäure/ Glutarsäure 5:1) enthält.

## Patentansprüche

1. Verfahren zur Herstellung von gegebenenfalls substituierten aliphatischen C₄-C₁₈-α,ω-Dicarbonsäuren durch Oxidation von gegebenenfalls substituierten C₆-C₂₀-α,ω-Dienen mit Wasserstoffperoxid oder Verbindungen, die unter den Reaktionsbedingungen Wasserstoffperoxid freisetzen, dadurch gekennzeichnet, dass die Reaktion in Gegenwart eines Übergangsmetallkatalysators der Gruppe VI und einer Mischung, welche mindestens eine organische Säure und mindestens ein aprotisches organisches Lösungsmittel enthält, durchgeführt wird.

2. Verfahren gemäß Anspruch 1, dadurch gekennzeichnet, dass als organische Säuren geradkettige oder verzweigte C₂-C₈-Alkylcarbonsäuren, C₆-C₁₄-Arylcarbonsäuren oder geradkettige oder verzweigte C₇-C₁₅-Arylalkylcarbonsäuren eingesetzt werden, die gegebenenfalls mindestens einen weiteren Substituenten des Typs Halogen, geradkettiges oder verzweigtes C₁-C₆-Alkyl, C₃-C₇-Cycloalkyl oder Carboxyl tragen.

3. Verfahren gemäß einem oder mehreren der vorangegangenen Ansprüche 1 und 2, dadurch gekennzeichnet, dass als organische Säure Essigsäure eingesetzt wird.

4. Verfahren gemäß einem oder mehreren der vorangegangenen Ansprüchen 1 bis 3, dadurch gekennzeichnet, dass als aprotisches organisches Lösungsmittel Dioxan, Ethylacetat, Propylacetat, Butylacetat, Chlorbenzol, Acetonitril oder Benzonitril eingesetzt wird.

5. Verfahren nach einem oder mehreren der vorangegangenen Ansprüche 1 bis 4, dadurch gekennzeichnet, dass das Mischungsverhältnis von mindestens einer organischen Säure und mindestens einem aprotischen Lösungsmittel 1:5 (v/v) bis 5:1 (v/v), bevorzugt 1:2 (v/v) bis 2:1 (v/v), besonders bevorzugt 1:1 (v/v) ist.

6. Verfahren nach einem oder mehreren der vorangegangenen Ansprüche 1 bis 5, dadurch gekennzeichnet, dass die als Edukte eingesetzten C₆-C₂₀-α,ω-Diene gegebenenfalls mindestens einen Substituenten, ausgewählt aus der Reihe C₁-C₆-Alkyl, C₆-C₂₂-Aryl, C₇-C₂₄-Arylalkyl oder Halogen tragen.

7. Verfahren nach einem oder mehreren der vorangegangenen Ansprüche 1 bis 6, dadurch gekennzeichnet, dass der Übergangsmetallkatalysator der Gruppe VI eine Wolframsäure, ein Wolframat oder ein Wolframkomplex ist.

8. Verfahren nach einem oder mehreren der vorangegangenen Ansprüche 1 bis 7, dadurch gekennzeichnet, dass 9 bis 20 Äquivalente Wasserstoffperoxid, bevorzugt 10 bis 13 Äquivalente, bezogen auf das eingesetzte C₆-C₂₀-α,ω-Dien, eingesetzt werden,

9. Verfahren nach einem oder mehreren der vorangegangenen Ansprüche 1 bis 8, dadurch gekennzeichnet, dass die Oxidation entlang eines Temperaturprofils erfolgt und die Äquivalente an Wasserstoffperoxid in zwei Stufen zugegeben werden.

10. Verfahren nach einem oder mehreren der vorangegangenen Ansprüche 1 bis 9, dadurch gekennzeichnet, dass durch Oxidation von 1,7-Octadien Adipinsäure hergestellt wird.
